# EUROPEAN PATENT APPLICATION

(11) **EP 2 418 273 A2**
(43) Date of publication of application: **15.02.2012**
(21) Application number: 10761921.5
(22) Date of filing: 03.03.2010
(51) Int. Cl.: C12N 5/10

(54) **METHOD FOR PRODUCING PLURIPOTENTIAL CELLS**

(30) Priority: 10.04.2009 RU 2009113457
(71) Applicant: Obschestvo S Ogranichennoi Otvetstvennostyu "Laboratoria Kletochnykh Tekhnologiy", Moscow 127051 (RU)
(72) Inventor: KISELEV, Sergey Lvovich, Moscow 123458 (RU); LAGAR'KOVA, Mariya Andreevna, Moscow 105318 (RU); SHUTOVA, Mariya Vladimirovna, Moscow 115419 (RU)
(74) Representative: Henrion, Oliver
(86) International application number: PCT/RU2010/000099
(87) International publication number: WO 2010/117301

(57) **Abstract**

The invention relates to biotechnology, and particularly to the preparation of pluripotent stem cells. The method involves introduction into umbilical cord and placental stem cells of RNA with at least one sequence which ensures the transition of cells to the pluripotent state. The method enables to effectively prepare pluripotent stem cells from the cells of mammalian placenta and umbilical cord which have not yet acquired somatic mutations, which reduces the risk of oncogenesis and other adverse effects of reprogramming.

## Description

### Field of Invention

This invention relates to biotechnology, and particularly to preparing pluripotent stem cells from the cells of mammalian placenta and umbilical cord. The pluripotent stem cells prepared as a result of the 'reprogramming' can be widely used in medicine and 'reprogramming' and `transdifferentiation'.

### Prior Art

In 2006, genes of four transcription factors were introduced in terminally differentiated mouse cells (fibroblasts) using retroviruses in Shinya Yamanaka lab (Kyoto, Japan) (1). After 16 days, researchers found that fibroblasts change their morphology, and behavior of cells in culture also changes. As a result of the selection, the cells which resembled mouse embryonic stem cells in properties and characteristics were left. They were able to differentiate into cells of an adult organism and colonize tissues of animals after their introduction into the blastocyst. In appearance, properties, and genetic portrait they were close but not identical to ESCs derived by natural means, so they were called iPS (induced pluripotency stem) cells. The combination of genes Oct3/4, Sox2, c-Myc, and Klf4 out of the 24 candidate genes gave the best results. The genes Oct3/4 and Sox2 encode transcription factors which function on the early stages of embryogenesis, and they don't work in adult cells. C-Myc gene also encodes a transcription factor, but unlike the previous two it is not specific, and is involved in controlling the cell cycle and division of any cell and is proto-oncogene. The last gene Klf4 too has no specific time or tissue specificity and encodes the transcription factor, may play a role both as a transcription activator and repressor, however, its functions are known very little. If the first two genes are highly specific for the early stages of embryogenesis, the latter two play an important role in the formation of tumors.

The use of proto-oncogene c-Myc led to an increased frequency of tumor formation in animals obtained, and the effectiveness of the procedure for obtaining the original cells was very low.

Today, iPS cells have been prepared from dozens of specialized tissues, including that of human. Apart from embryonic and dermal fibroblasts, neuronal precursors, intestinal epithelial cells, hematopoietic, mesenchymal stem cells, myocytes, keratinocytes, hepatocytes, elementary and other cells were transformed into iPS (2).

Despite similar experiments in different laboratories, the most efficient of them is the pioneer genetic system (1), all others are not as effective.

Strong mutagenic impact (viruses + transcription factor genes + oncogenes) along with the selection on the ESC cultivation medium enables some single cells to get over the selection process. The iPS cell formation efficiency recorded in the initial research was up to some basis points. It must be the reason for the wide scatter observed in 'reprogrammed' clones. Such reasoning indeed supports the idea that cells, which are closer in their properties to pluripotent stem cells as compared to the majority, may potentially exist in an adult organism.

Disadvantages of all the existing methods used for the somatic cell reprogramming include:
1. Injection of the gene as a part of various vectors - especially viral ones - leads to the transgenic cell material, where genes and vectors that contain genes integrate into different parts of the genome, thus modifying the organism, which may lead to the unwanted consequences, including oncogenesis, in future. Apart from the oncogenesis caused by the incorporation of the viral DNA into the genome, there is a considerable risk of transgene (gene introduced as a part of a virus) reactivation in the process of cell activity and further cell differentiation. This may lead to another reprogramming of a single cell, and it may acquire pluripotent properties. At the same time, if the reprogrammed cells are surrounded by previously differentiated cells of the organism, they may invest into other tissues, which will lead to the emergence of choristias and hamartias. This improper development of a tissue may finally lead to the formation of a tumor. Genetic modification using DNA has a range of disadvantages related to the productive efficiency of the proteins coded with DNA. First, RNA should be synthesized from a DNA molecule. As the RNA synthesis takes place in the nucleus, a DNA molecule should penetrate from the cytoplasm into the nucleus. The majority of DNA delivery systems, excluding the viral one, deliver DNA in the cytoplasm only, and then it should occasionally penetrate into the nucleus. At the same time, the exact number of molecules that have penetrated the nucleus is not known or standard, and the scatter may lead to adverse consequences, when used in practice. Second, the RNA synthesis in a cell requires a promoter (a sequence that enables entry of the cell transcription factors). Different types of cells have different sets of transcription factors, which makes it difficult to select a promoter sequence that will enable standard expression from the introduced construction in the cell nucleus. E.g., the EF1 alpha promoter does not work in fibroblasts. Viral promoters CMV, RSV, SV40 and others are the most universal promoter sequences that work in the majority of cells, but have different efficiency. At the same time, there is no standard work for different types of cells and a part of viral genome is introduced into DNA, which may possibly lead to oncogenesis in cases when the promoter sequence integrates into the cell genome. Third, RNA synthesized in the cell nucleus should migrate into the cytoplasm. In case of the endogenous RNA synthesis from a genome sequence, molecules carry introns and other sequences that enable RNA stability and its directed transport into the cytoplasm. In the majority of cases when a transgene is used, RNA does not have such sequences. Due to this fact, the efficiency of the RNA transport from the nucleus to the cytoplasm is low, and unprotected RNA sequences are exposed to hydrolysis by ribonucleases. This leads to the considerable decrease in efficiency and absence of any standardization in the protein synthesis in the cytoplasm.
2. During individual development of the organism (ontogenesis), cells go through totipotency (zygote), pluripotency (inner cell mass, embryonic stem cells in vitro), and terminal differentiation (specialized tissues). Specialized tissues of the organism and regional stem cells, that support angiogenesis of these tissues, exist within a finite period of time. The ageing process is followed by the death of the organism. The ageing process is connected to the range of intracellular events, including the accumulation of biological 'trash' (undecomposable proteins, lipids, etc.) and genetic 'trash' (mutations accumulated in the course of ontogenesis) in the cells. Thus, during ontogenesis cells of an adult organism accumulate irreversible changes that cause the ageing process and lead to the death of the organism. The genetic program of a cell in an adult organism may be changed by a range of methods, including reprogramming using nucleus transfer (Wilmut) and genetic reprogramming using genes (Weintraub, Yamanaka). However, in these cases the reprogramming is performed in the genome and cells that have already accumulated irreversible changes. Further use of such a material in therapy may lead to adverse consequences - premature ageing, death, or oncogenesis at the best. Besides, sometimes mammalian and human cells necessary for the reprogramming are not available, while its sampling is usually invasive.

### Brief figure description.

**Fig.1** Endotheliocyte culture.
**Fig.2** Colonies of selected somatic pluripotent stem cells.
**Fig.3** Colonies of selected somatic pluripotent stem cells on the mTeSR medium.
**Fig.4** Karyotype of the cells with induced pluripotency.
**Fig.5 (A)** 8-day old embryoid bodies. **(B-D)** Immunohistochemical analysis of the 14-day old embryoid bodies. **(E,F)** Hematoxylin-eosin staining with terat.
**Fig.6** Neuroepithelium.

### Disclosure of the invention

As a result of the carried out research, it was found that pluripotent stem cells may be efficiently prepared from the cells of mammalian placenta and umbilical cord by injecting them with RNA that contains sequences enabling the transition of cells to the pluripotent state.

The suggested method of somatic cell reprogramming is based on the injection of RNA obtained in the transcription system in vitro (i.e. outside the cell) into the cell. To do so, safe prokaryotic promoter sequences, e.g. SP6, T7, T3 and others, and respective polymerases - SP6, T7, T3 - shall be used. RNA synthesized in vitro shall be purified with up-to-date ion-exchange chromatography methods from foreign proteins and foreign DNA, before it is introduced into the cell. Quantity of RNA obtained in vitro may be measured in milligrams and even grams, while the transcription system in vitro may be standardized. In the latter case, it is possible to calculate the RNA quantity accurate to a molecule that codes a protein and may penetrate into the mammalian cell in vitro or in vivo. Such methods as chemical transfection with chemicals, e.g. dendrimers, biological transfection with lipids and proteins, e.g. liposomes, electromechanic transfection, e.g. electroporation. Besides, RNA may be introduced as a part of bacteria, cationic and anionic polymers, liposome complexes, exposed to the electric field, inert particles, or other methods applicable to the introduction of nucleic acids into the cell. In this case RNA gets into the cell cytoplasm without leaving the nucleus and degradation. Thus, it may be immediately involved in the synthesis of respective proteins in the cytoplasm. The quantity of RNA involved in the synthesis may be accurately calculated. Average half-decay time of an RNA molecule in the cell is up to several hours (3-6), and the injected molecule is deprived of the sequences that may be involved in the DNA synthesis. Thus, when RNA is injected, the cell is not modified on the genetic level, the cell genome does not change, and the cell does not obtain genetic elements of viruses that may cause oncogenesis. Besides, no extra time is required for molecules to penetrate into the nucleus, for the RNA synthesis and for its transportation back to the cytoplasm, while the quantity of injected RNA may be standardized.

Unlike other methods of preparing pluripotent stem cells, the current method uses cells of mammalian placenta and umbilical cord that contain particular sequences (as parts of RNA) enabling the transition of cells to the pluripotent state.

When a mammal (a human being) is being born, the placenta and umbilical cord get separated from it. The placenta and umbilical cord contain a range of cell populations, e.g. fibroblasts, keratinocytes, endotheliocytes, hemopoietic and mesenchymal cells that may be isolated, multiplied, and stored. These cells are the youngest cells in the organism, they have never been exposed to the environment and at this stage may be obtained without any invasion. Thus, cells of all types contained in placenta and umbilical cord are the most accessible and the safest for individual reprogramming, as they have a minimum set of defects acquired in the course of ontogenesis. All cells selected from placenta and umbilical cord may be used for the introduction of ribonucleic acids of a particular composition to shift them to the pluripotent state.

Reprogramming factors that correspond to proteins Oct4 (POU5F1),Sox2, HNF3b, PDX1, HNF6, ngn3, PAX4, NKX 2.2,FoxB3,HNF4a, Nkx2.5, Nkx2.2, Nkx6.1 and other families of Nkx, Pax4, Klf4, Ngn3, Pdx1, Mafa or others that cause the necessary changes of the culture properties may be used as sequences enabling the transition of cells to the pluripotent state. They may be introduced into the cells as part of RNA simultaneously or one at a time.

Thus, the invention may be characterized by the following essential features: "The method of preparing pluripotent stem cells by introducing into mammalian cells nucleic acids with least one sequence which ensures the transition of cells to the pluripotent state is **characterized in that** in this method use of cells of placenta and umbilical cord is made, and RNA is introduced as nucleic acids."

For further use, pluripotent stem cells obtained in such a way may be selected in 2-6 days using media that maintain the growth of mammalian and human embryonic stem cells (KnockOut DMEM, serum substitute, mTeSR or others) under the absence or presence of the feeder cell layer. To prepare the necessary cell phenotype, media maintaining the growth of muscle cells, e.g. cardiomyocytes, secretory cells such as insulin producing cells, filter cells such as hepatocytes, neuronal cells, glia cells, and others, may be used. If pluripotent stem cells are cultivated on these media, cells of the necessary phenotype form in 10-20 days. Obtained types of cells may be multiplied in vitro to increase their quantity. Particular quantities of cells may be used for further application in the disease therapy and substance screening.

### Embodiment of invention

Possibility of the embodiment of invention is affirmed by the following example.

### Example 1.

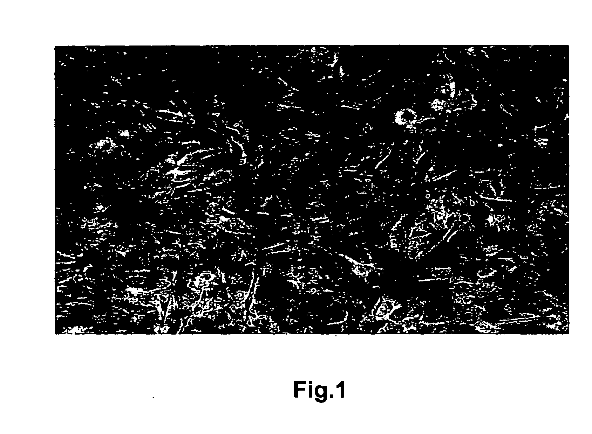

Endotheliocytes separated from a human umbilical cord were cultivated in a particular medium composition, including: alpha MEM, 20% FBS, nonessential amino acids,
hydrocortisone - 10⁻⁶ M , IGF - 5 ng/ml, bFGF - 5 ng/ml, VEGF - 10 ng/ml, EGF - 5 ng/ml (Fig. 1).

The transfection of endotheliocytes culture with the RNA synthesized in vitro was performed at the 2^{nd}-3^{rd} passage of cultivation. The Turbofect (Fermentas) reagent was used for the delivery in compliance with the manufacturer's guidelines. RNA-containing sequences corresponding to proteins Oct4 (POUSF1), Sox2, and Klf4 were used. Transfections were performed daily for 3 days. After 6 days since the last transfection, the cells were moved to other bowls covered with 0.1 % gelatin (Merck) and containing inactivated embryonic fibroblasts of a mouse as the feeder. Composition of the culture medium in use was the following: 80% KnockOut DMEM, 20% FBS (ES qualified), glutamine - 1 mM, 1% nonessential amino acids, penicillin - 50 units/ml, streptomycin - 50 µg/ml (all by Invitrogen), β-mercaptoethanol - 0.1 mM (Sigma).

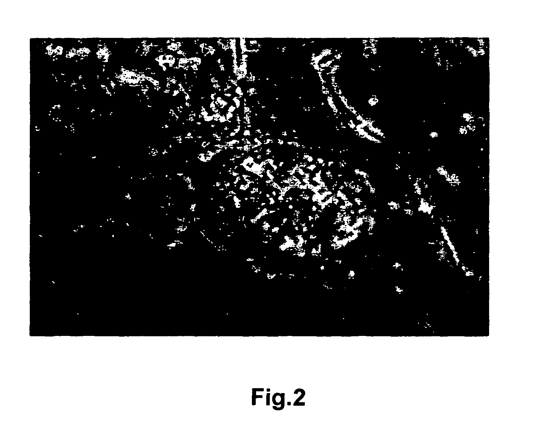

After 17 days colonies, identical to those formed by human embryonic cells, formed (Fig. 2).

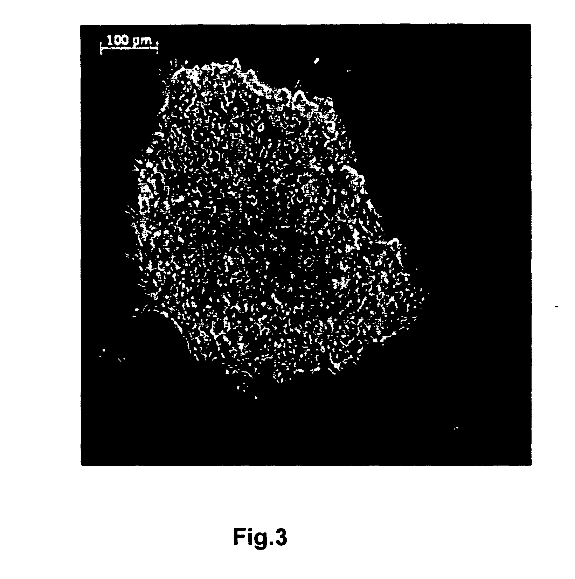

The formation of colonies also takes place in another medium maintaining the growth of embryonic stem cells, e.g. mTeSR (Fig. 3).

These cells may be called SSPC - selected somatic pluripotent stem cells.

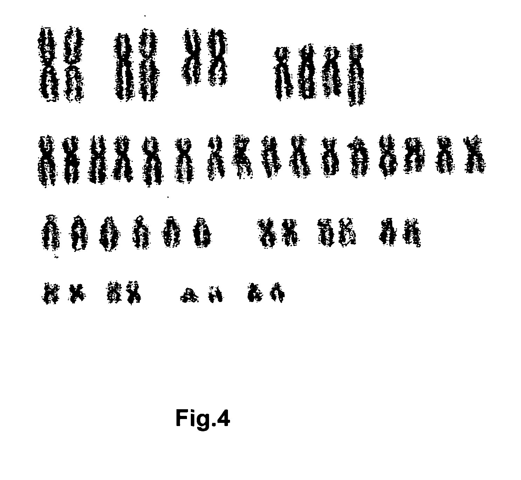

Efficiency of the SSPC preparing is over 0.001%. Obtained cell populations may be cultivated and multiplied under particular conditions. At the same time they retain regular phenotype corresponding to the initial cells (Fig. 4), but their phenotype and properties change. The use of sequences Oct4 (POU5F1), Sox2, and Klf4 leads to the emergence of markers SSEA4, TRA1-60, expression of genes Nanog, FoxD3 and others, which is characteristic of human embryonic stem cells.

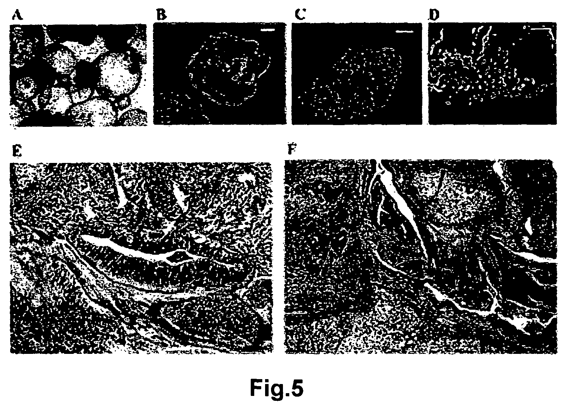

Obtained cells correspond to the initial ones in their genotype, including the immuno-histocompatibility complex of the 1^{st} and 2^{nd} classes. Obtained pluripotent stem cells may form tissues of all the three germ layers, which is supported by the formation of embryoid bodies (Fig. 5A).

Embryoid bodies contain cells of the three germ layers, mesoderm (Fig. 5B antibody staining against CD105 (green) and MOC-31 (red)), endoderm (Fig. 5D antibody staining against alpha-fetoprotein), ectoderm (Fig. 5C antibody staining against desmin), and teratomas (Fig. 5E, F hematoxylin-eosin staining with terat).

### Industrial applicability

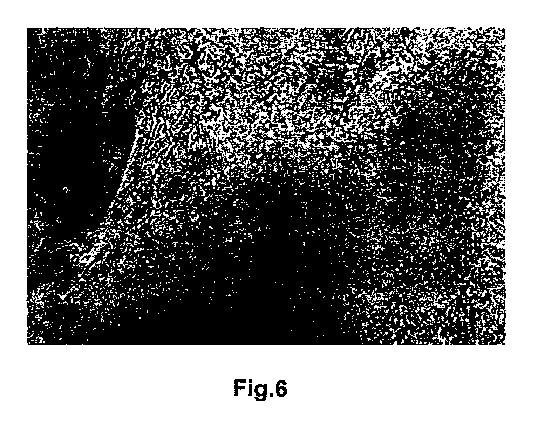

Thus, cells obtained from endothelium are pluripotent and may be used to prepare all types of mammalian (human) tissues. For example, Fig. 6 demonstrates neuroglia cells obtained from SSPC. Cells of a different specialization, including endothelium, cardiomyocytes, hepatocytes, beta cells, keratinocytes, sensorineural cells, pigmented cells and other cells of a mammalian organism may be also obtained. Such cells are genetically identical to the organism used as a source of the initial endotheliocytes.

The present example demonstrates only a particular case of preparing pluripotent stem cells.

The suggested method enables to effectively prepare pluripotent stem cells from the "young" cells of placenta and umbilical cord, which reduces the risk of oncogenesis and other adverse effects of reprogramming.

### Literature

1. Takahashi K Yamanaka S, Induction of Pluripotent Stem Cells from Mouse Embryonic and Adult Fibroblast Cultures by Defined Factors 2006, Cell, 126, 663-676
2. Wernig, M., Lengner, C.J., Hanna, J., Lodato, M.A., Steine, E., Foreman, R.,Staerk, J., Markoulaki, S., and Jaenisch, R. (2008a). A drug-inducible transgenic system for direct reprogramming of multiple somatic cell types. Nat. Biotechnol. 26, 916-924.

## Claims

1. A method of preparing pluripotent stem cells by introducing into mammalian cells nucleic acids with least one sequence which ensures the transition of cells to the pluripotent state, **characterized in that** cells of mammalian placenta and umbilical cord are employed, and RNA is introduced as nucleic acids.
